Europäisches Patentamt

(19) European Patent Office

Office européen des brevets

(11) Numéro de publication: **0 088 656**
**B1**

(12) **FASCICULE DE BREVET EUROPEEN**

(45) Date de publication du fascicule du brevet:
13.11.85

(51) Int. Cl.⁴: **C 12 P 7/28,** C 12 P 7/16,
C 12 N 1/36, C 12 N 1/20 //
C12R1/145, C12N15/00

(21) Numéro de dépôt: 83400339.4

(22) Date de dépôt: 18.02.83

(54) Obtention de mutants de clostridium acétobutylicum à haute productivité de butanol et d'acétone, mutants obtenus et utilisation de ces mutants pour la production conjointe de butanol et d'acétone.

(30) Priorité: 08.03.82 FR 8203980

(43) Date de publication de la demande:
14.09.83 Bulletin 83/37

(45) Mention de la délivrance du brevet:
13.11.85 Bulletin 85/46

(84) Etats contractants désignés:
AT BE DE GB IT NL SE

(56) Documents cités:
MICROBIAL TECHNOLOGY, vol. 1, 1979, pages 187-209,
Edited by H.J. Peppler et al., Academic Press, New York;
M.T. WALTON et al.: "Production of Butanol-Aceton by
Fermantation"
INDUSTRIELLE MIKROBIOLOGIE, 2éme édition, 1980,
pages 291-305, Springer Verlag Berlin (DE); H.J. REHM:
"Butanol-aceton und weitere primäre Produkte aus
Clostridien"
INDUSTRIELLE MIKROBIOLOGIE, 2ème édition, 1980,
pages 48-64; Springer Verlag Berlin (DE); H.J. REHM:
"Mikroorganismengenetik unter industriellen
Gesichtpunkten"

(73) Titulaire: **INSTITUT FRANCAIS DU PETROLE, 4, Avenue
de Bois-Préau, F-92502 Rueil-Malmaison (FR)**

(72) Inventeur: **Hermann, Monique, 5, rue Fragonard,
F-75017 Paris (FR)**
Inventeur: **Fayolle, Françoise, 37, rue Galléni Batiment A,
Escalier 3, F-92240 Maladkoff (FR)**
Inventeur: **Marchal, Rémy, 70, rue des Cormiers,
F-78400 Chatou (FR)**

BUNDESDRUCKEREI BERLIN

## Description

La fermentation acétonobutylique, connue depuis longtemps, permet de produire, à partir de sucres divers, un mélange de produits d'intérêt industriel, le butanol, l'acétone et l'éthanol (ABE), et constitue un moyen particulièrement efficace d'obtenir le produit principal, le n-butanol. L'intérêt de cette voie d'obtention de composés entrant dans la composition de carburants de substitution a, par ailleurs, été récemment mise en évidence (demandes de brevets français 80/12822 et 80/17147 (FR-A-2 483 944/2 487 846/2 488 272) de l'Institut Français du Pétrole). La limitation principale de cette fermentation a cependant été jusqu'à présent, la faiblesse relative de la concentration des produits formés qui ne dépasse généralement pas, dans les meilleures conditions, une concentration totale de 20 g/l, typiquement 10—13 g/l de n-butanol, 4 à 7 g/l d'acétone, 0 à 2 g/l d'éthanol, comme indiqué dans les articles de synthèse de M. T. Walton et J. L. Martin (Microbiol fermentation 2nd edition, vol. 1, Academic Press, 1979, pp. 187—209) et de S. C. Prescott et C. G. Dunn (Industrial Microbiology, Mc Graw Hill, 1959, pp. 250—293).

L'objet de la présente invention est de décrire une méthode d'obtention de mutants de Clostridium acetobutylicum, qui, cultivés dans des conditions adéquates, permettent d'obtenir des concentrations en ABE largement supérieures à celles fournies par la souche mère et dépassant 20 g/l. De tels mutants sont dénommés ici mutants hyperproducteurs d'ABE.

L'utilisation des techniques habituelles de mutagénèse s'est heurtée dans le cas de Clostridium acetobutylicum, à plusieurs difficultés. La première réside dans le caractère anaérobie stricte de cette bactérie. On ne peut en effet obtenir un développement normal de colonies sur milieu nutritif solide, technique de base utilisée en microbiologie pour l'isolement, la sélection et la purification de souches de microorganismes, qu'en utilisant des précautions particulières visant à éliminer toutes traces d'oxygène dans les manipulations effectuées. Les manipulations ont donc été effectuées dans une »boîte à gants« anaérobie avec des milieux préalablement réduits, par exemple dans les conditions décrites par L. V. Holdeman, E. P. Cato et W. E. C. Moore (Anaerobe Laboratory manual, 4th Edition, 1977, Virginia Polytechnic Institute, Blacksburg, Va, p. 144).

Plusieurs techniques de mutagénèse ont été utilisées dans les conditions qui viennent d'être mentionnées. On a d'une part essayé plusieurs agents mutagènes notamment l'éthylméthylsulfonate (EMS) et la N-méthyl N'-nitro N-nitrosoguanidine (NG), l'ICR 191, l'acide nitreux, le nitroquinoline-N-oxyde. On a d'autre part, fait varier la mise en oeuvre de ces agents mutagènes. La technique la plus habituelle en la matière consiste à mutagénéiser milieu liquide une suspension microbienne en l'exposant à une concentration donnée, pendant un temps déterminé, à l'agent mutagène étudié. On centrifuge ensuite et on lave la suspension microbienne pour éliminer l'agent mutagène, on la remet en culture puis on étale cette suspension sur milieu solide nutritif (boîtes de Pétri) pour obtenir des colonies individuelles de microorganismes survivants et on sélectionne ensuite parmi ces colonies, les mutants présentant les caractéristiques désirées. En faisant varier le temps de mutagénèse et la concentration de l'agent mutagène, on fait ainsi varier le taux de mutation et de mortalité imposé à la population de microorganismes étudiée et on sélectionne les conditions qui fournissent le taux de mutation le plus favorable. Cette technique bien connue est décrite dans des ouvrages spécialisés, notamment par J. H. Miller (Experiments in molecular genetics. Cold Spring Harbor, 1972, p. 125).

On a constaté, avec tous les agents mutagènes étudiés, que l'utilisation de cette technique ne fournissait qu'un taux très réduit de mutants et en aucun cas des mutants hyperproducteurs d'ABE.

La technique de mutagénèse de l'invention consiste à effectuer en une seule étape en milieu solide la mutagénèse dans une gamme de concentration variée et la sélection des mutants recherchés. On a pour cela effectué des étalements, sur un milieu nutritif solide, renfermant du n-butanol, par exemple dans des boîtes de Pétri, de cultures liquides en croissance de Clostridium acetobutylicum. Pour obtenir un gradient de concentration en agent mutagène, on a disposé en un point, par exemple au centre de chaque boîte, l'agent mutagène, de préférence la nitrosoguanidine (NG) créant ainsi le gradient recherché par diffusion progressive du mutagène. Pour effectuer la sélection de mutants hyperproducteurs d'ABE, on recherche des mutants résistant au butanol dans l'hypothèse où ces derniers supporteraient une accumulation plus importante de butanol lors des essais de production d'ABE. On a donc ajouté le n-butanol aux boîtes de Pétri qui viennent d'être décrites à des concentrations différentes d'une boîte à l'autre, mais de plus en plus importantes, toutes inhibitrices de la souche mère. Après incubation, dans les conditions connues d'incubation de C. acetobutylicum, ces boîtes de Pétri ont fourni des colonies isolées de mutants résistant au n-Butanol. On a examiné les capacités de production d'ABE de ces mutants et constaté que la plupart étaient surproducteurs d'ABE. Lorsque des concentrations adéquates de butanol étaient utilisées dans les boîtes de Pétri, de nombreux mutants ont été ainsi obtenus avec diverses souches de C. acetobutylicum. D'une façon générale, la technique s'est révélée reproductible. Certaines souches de C. acetobutylicum cependant ne fournissent pas naturellement de colonies isolées des suffisamment ponctuelles et auc contours distincts. Dans ce dernier cas, l'obtention de mutants a nécessité l'isolement de variants spontanés formant des colonies aux contours distincts pour se prêter commodément aux manipulations décrites plus haut.

Les raisons de l'efficacité de la technique qui vient d'être décrite pour l'isolement de mutants hyperproducteurs d'ABE, ne sont pas complètement comprises. On peut penser que cette technique qui ne comporte que des manipulations qui peuvent être effectuées en chambre anaérobie se prête mieux à des conditions d'anaérobiose pousse. On remarque aussi que le test de sélection (crible) choisi, celui de résistance au butanol, est un bon crible pour la sélection de mutants hyperproducteurs d'ABE, ce qui indique que ces deux propriétés sont liées. En ce qui concerne l'importance de l'agent mutagène utilisé, il faut noter que les agents mutagènes ne sont pas équivalents et que, par exemple, l'emploi de l'EMS, dans les mêmes conditions, a donné des résultats nettement moins favorables que ceux obtenus avec la nitrosoguanidine. Les mutants faisant l'objet de la présente invention ont donc été obtenus en utilisant, comme test de sélection, la résistance au butanol après un protocole de mutagénèse particulier. Ces mutants ont pu être obtenus à partir de différentes souches-mères de Clostridium acetobutylicum, en particulier des souches précédemment isolées au laboratoire à partir de milieu naturels. Il sont caractérisés par leur résistance au butanol (et autres solvants) ainsi que par leur production de solvants (butanol et acétone) qui sont supérieures à celles de la souche-mère.

On à également remarqué que les mutants obtenus présentaient des caractères de sporulation différents de ceux de la souche-mère, en ce sens que la proportion de la population bactérienne susceptible de former des spores en fin de culture était plus faible chez les mutants que chez la souche sauvage.

Les exemples qui suivent illustrent le protocole d'obtention des mutants et leur caractérisation dans le cas d'une souche isolée au laboratoire (souche IFP 903) et utilisée pour la fermentation sur jus de topinambour. L'obtention de mutants et leurs performances ne sont pas liées à l'utilisation d'un substrat sucré particulier et ont été observées avec les divers sucres utilisables par Clostridium acetobutylicum notamment glucose, fructose, saccharose, amidon, cellobiose, xylose et hydrolysats de matériaux lignocellulosiques tels que les tiges de maïs.

Les caractéristiques de la souches IFP 903 qui ont conduit à l'identifier comme une souche de Clostridium acetobutylicum sont données ce-dessous. Les mutants obtenus de la façon décrite plus haut et illustrés dans l'exemple 1, présentent les mêmes caractéristiques taxonomiques que la souche-mère sauf, comme mentionné plus haut, un taux de sporulation inférieur à celui de la souche-mère.

## CARACTERES DE LA SOUCHE IFP 903 DE CLOSTRIDIUM ACETOBUTYLICUM

Morphologie:
    batonnets à bouts ronds, immobiles, isolès, Gram positifs avec spores ovales fréquemment subterminales, occasionnellement terminales, sporange gonflé.

Culture:
    bouillon PY-glucose (milieu PY décrit par Miller, déjà cité): fort trouble avec gaz,
    bouillon PY: faible trouble sans gaz,
    gélose (PY-glucosé): colonies rondes blanches de 1—2 mm de diamètre, jaunissant en vieillissant.

La croissance s'effectue dans des conditions d'anaérobiose stricte. Les sucres utilisés sont principalement transformés en butanol et en acétone (et un peu d'éthanol). Des acides volatils (acides acétique et butyrique) sont formés en plus faibles quantités. Les gaz formés sont constitués de gaz carbonique et d'hydrogène.

Glucides et autres substrats carbonés:
    Croissance vigoureuse avec production de gaz et d'acide sur:
    raffinose, lactose, mélibiose, lévulose, maltose, tréhalose, cellobiose, mannose, xylose, salicine, galactose, glucose, $\beta$-méthylglucoside, amidon, saccharose, mannitol.
    Croissance moins vigoureuse avec production de gaz et d'acide sur:
    inositol, esculine, $\alpha$-méthylglucoside.
    Croissance peu vigoureuse, peu de gaz et d'acide:
    sorbitol, rhamnose.
    Non utilisés:
    érythritol, arabinose, ribose, glycérol, pectine.

Protéines:
    lait: coagulé; caséine; non utilisée; sérum: non utilisé; gélatine: non utilisée.

Lipides:
    lécithinase: résultat négatif; tributyrine: pas de culture.

Autres caractères:
    hémolyse: négative, urée: négative ou positive très faible, sulfites réduits, production de $SH_2$: positive; nitrates: non utilisés; indole: non formé; acétoïne: non produite.

L'ensemble de ces caractères comparés à ceux présentés par Mc Coy et coll. (E. E. B. Mc Coy, E. B. Fred, W. H. Pterson et E. G. Hastings, Journal of Infectious Diseases — 1926 — 39, 457—483) pour Clostridium acetobutylicum conduissent à classer la souche IFP 903 et les souches obtenues à partir de cette dernière par le protocole décrit plus haut comme appartenant à l'espèce Clostridium acetobutylicum.

## Exemple 1

### 1. Protocole d'obtention de mutants résistants au butanol de Clostridium acetobutylicum

Une souche de Clostridium acetobutylicum (IFP n° 903) à été mise en croissance durant une nuit en chambre anaérobie sur un milieu complet, le milieu PY décrit par Miller, déjà cité. La source de carbone était du glucose à raison de 10 g/l.

La souche a été remise en croissance le lendemain matin dans 10 ml de milieu PY frais. Le même jour, des boîtes de milieu PY gélosé on été préparées dans la chambre anaérobie avec des concentrations croissantes de n-butanol: 5, 7, 10 et 12 grammes par litre. Après croissance de la souche (8 h), 0,1 ml de culture a été étalé sur chaque boîte. 15 minutes après, un cristal de nitrosoguanidine était déposé au centre de chaque boîte. Les boîtes ont été mises à incuber à 32°C pendant 3 jours dans la chambre anaérobie.

On a pu observer l'action inhibitrice partielle de la nitrosoguanidine sur les boîtes contenant 5, 7 et 10 g par litre de butanol. Aucune croissance n'était observée sur la boîte contenant 12 grammes par litre de butanol. Sur les boîtes contenant 5 et 7 grammes par litre, une croissance uniforme de la souche était observée au-delà de la zone toxique. Sur la boîte contenant 10 grammes par litre, des mutants résistant formaient une auréole et 11 d'entre eux ont été purifiés par deux passages successifs sur boîte contenant 10 g par litre de n-butanol. Avec cette concentration en n-butanol, aucune croissance de la souche sauvage d'était observée sur boîte dans les conditions expérimentales utilisées.

### 2. Détermination de la concentration minimale inhibitrice (CMI)

La résistance au n-butanol est une caractéristique importante des mutants obtenus comme le démontre l'expérience décrite ci-dessous. Dans cette expérience, comme certaines manipulations ne sont pas aisément réalisables dans la chambre anaérobie, on utilise alors, hors de la chambre, des milieux préréduits préparés dans cette dernière. Les milieux de culture habituels décrits plus haut sont préparés selon un protocole un peu différent. On prépare le milieu, on ajoute un indicateur de réduction, la résazurine hors de la chambre anaérobie, puis le milieu est chauffé jusqu'à décoloration de la résazurine et le milieu est alors introduit dans la chambre anaérobie, il est réparti dans des fioles en verre bouchées par un bouchon en butyl, puis une capsule en métal est scellée avec une pince. On sort alors les fioles de la chambre et on peut les utiliser telles quelles après stérilisation. En effet, on dispose d'un milieu sous atmosphère anaérobie dont on contrôle l'état réduit par la couleur de la résazurine.

Ces milieux prédéduits sont ensemencés à la seringne à travers le bouchon butyl; tous les prélèvements et toutes les additions se font de cette manière.

Ces milieux préréduits pont servi, entre autre, pour mesurer les concentration minimales inhibitrices (CMI) des différentes souches de mutants et de la souche-mère de C. acetobutylicum pour le butanol. Ce produit assez volatil ne subit pas d'évaporation dans les fioles scellées et on a ainsi une mesure exacte de la CMI.

On ajoute le butanol dans les fioles à des concentrations variées à l'aide d'une seringue. De la même façon, la souche à tester est introduite dans la fiole contenant 5 ml de milieu à raison de 0,1 ml d'une culture de 12 heures. Après 24 h d'incubation à 32°C, la lecture de la CMI est effectuée.

Les résultats sont résumés dans le tableau 1 (par la CMI, on mesure la toxicité d'un produit sur une souche bactérienne donnée, cette CMI correspond à la première concentration de ce produit pour laquelle on n'observe plus de croissance bactérienne).

| Souches | Concentration en butanol (en g/l) | | | | | | |
|---|---|---|---|---|---|---|---|
| | 0 | 5 | 10 | 13 | 15 | 18 | 20 |
| Témoin = souche-mère | + | + | + | — | — | — | — |
| Mutant n° 1 | + | + | + | + | — | — | — |
| Mutant n° 2 | + | + | + | + | + | — | — |
| Mutant n° 3 | + | + | + | + | + | — | — |
| Mutant n° 4 | + | + | + | + | + | + | — |
| Mutant n° 5 | + | + | + | + | + | + | — |
| Mutant n° 6 | + | + | + | + | — | — | — |
| Mutant n° 7 | + | + | + | + | — | — | — |
| Mutant n° 8 | + | + | + | + | — | — | — |
| Mutant n° 9 | + | + | + | + | — | — | — |
| Mutant n° 10 | + | + | + | + | + | — | — |
| Mutant n° 11 | + | + | + | + | + | — | — |

On voit ainsi qu'en général les souches mutantes sont plus résistantes au butanol que cette dernière. L'expérience décrite ci-dessous démontre que les mutants résistant au n-butanol obtenus sont également meilleurs producteurs d'ABE que le souche-mère.

### 3. Production de solvants par les mutants obtenus

L'excrétion de solvants a été déterminée dans des tubes contenant 10 ml d'un milieu synthétique aqueux, contenant par litre: $(NH_4)_2SO_4$, 3 g; $K_2HPO_4$, 0,5 g; $MgSO_4$, 7 $H_2O$, 10 mg; $MnCl_2$, 4 $H_2O$, 10 mg; $(NH_4)_2Mo_7O_{24}$, $H_2O$, 10 mg; $CaCO_3$, 3 g; extrait de levure, 4 g; glucose, 60 g. Le substrat utilisé était donc le glucose. Les cultures étaient effectuées dans les conditions classiques de stérilité et en anaérobiose (manipulations et incubation 3 jours à 32°C en chambre anaérobie).

Les résultats sont indiqués dans le tableau 2 suivant:

Tableau 2

Production de solvants par la souche IFP 903 des Clostridium acetobutylicum et ses mutants

|  | Acétone $g \times 1^{-1}$ | Butanol $g \times 1^{-1}$ | Solvants totaux $g \times 1^{-1}$ |
|---|---|---|---|
| Souche-mère (IFP 903) | 2,6 | 7,8 | 10,4 |
| Mutant n° 1 | 5,0 | 11,6 | 16,6 |
| Mutant n° 2 | 3,0 | 9,1 | 12,1 |
| Mutant n° 3 | 3,9 | 10,2 | 14,1 |
| Mutant n° 4 | 4,4 | 10,6 | 15,0 |
| Mutant n° 5 | 3,9 | 10,7 | 14,6 |
| Mutant n° 6 | 4,1 | 10,5 | 14,6 |
| Mutant n° 7 | 4,4 | 12,3 | 16,7 |
| Mutant n° 8 | 4,4 | 11,3 | 15,7 |
| Mutant n° 9 | 3,3 | 9,4 | 12,7 |
| Mutant n° 10 | 4,8 | 12,5 | 17,3 |
| Mutant n° 11 | 4,1 | 12 | 16,1 |

## Exemple 2

Production de solvants en fermenteur par les mutants obtenus. La souche sauvage de C. acetobutylicum (IFP 903) et les mutants obtenus sont cultivés dans des fermenteurs de type BIOLAFITTE de 6 litres de capacité, contenant 4 litres d'un milieu constitué par un jus de pressage de topinambour dilué au demi, hydrolysé chimiquement à l'acide sulfurique puis neutralisé à la chaux à pH 7,0. Le milieu contient en outre 3 g.l$^{-1}$ de $(NH_4)_2SO_4$ et 5 g.l$^{-1}$ de $CaCO_3$. L'acticité fermentaire est mesurée par l'importance du dégagement gazeux sur un compteur volumétrique et par la composition du milieu final en solvants, déterminée par chromatographie en phase gazeuse. Le tableau ci-dessous rassemble les résultats obtenus par la souche sauvage et par 4 mutants hyperproducteurs après une fermentation d'une durée de 36 heures à 34°C, conduite dans les conditions standard de stérilité et en anaérobiose (milieux désaérés par chauffage et maintenus sous atmosphère inerte).

| Souche (a) | Acétone $g \times 1^{-1}$ | Butanol $g \times 1^{-1}$ | Solvants totaux $g \times 1^{-1}$ | Gaz 1/1 de milieu |
|---|---|---|---|---|
| Souche-mère IFP 903 | 4,5 | 10,5 | 15,0 | 21,0 |
| Mutant n° 8 (IFP 904) | 10,0 | 15,0 | 25,0 | 37,0 |
| Mutant n° 5 | 9,0 | 13,6 | 22,6 | 33,5 |
| Mutant n° 4 | 8,0 | 13,4 | 21,4 | 31,5 |
| Mutant n° 11 | 8,4 | 13,3 | 21,7 | 32,5 |

(a)   le mutant n° 8 a reçu le numéro 904 dans la collection IFP.

Les souches IFP 904 ont été déposées le 4 mars 1982 à l'American Type Culture Collection (ATCC). Elles ont reçu les numéros respectifs ATCC 39.057 et 39.058.

**Revendications**

1. Procédé d'obtention de mutants de Clostridium acetobutylicum à haute productivité de n-butanol et d'acétone et à résistance améliorée au n-butanol, qui consiste, dans des conditions anaérobies:

à étaler une culture aqueuse d'une souche de Clostridium acetobutylicum à la surface d'un milieu solide de culture renfermant du n-butanol,
à disposer un agent mutagène à la surface du milieu de culture,
à soumettre le milieu résultant à des conditions de développement de ladite souche,
et à recueillir au moins une partie d'une colonie de mutants formée,
la concentration du n-butanol dans le milieu de culture étant au moins égale à celle à partir de laquelle on n'observe plus de développement substantiel de la souche de départ, en l'absence d'agent mutagène, mais inférieure à celle à partir de laquelle, en présence de l'agent mutagène, aucune colonie de développment ne se forme.

2. Procédé selon la revendication 1, dans lequel l'agent mutagène est la N-méthyl N'-nitro N-nitrosoguanidine.

3. Procédé selon la revendication 1 ou 2, dans lequel le mutant recueilli est purifié en le mettant à nouveau en culture au moins une fois dans un milieu de culture renfermant du n-butanol en concentration supérieure à la concentration minimale pour laquelle la souche non mutée ne se développe plus mais inférieure à la concentration minimale pour laquelle le mutant ne se dévewloppe plus.

4. Procédé selon l'une des revendications 1 à 3, dans lequel l'agent mutagène est disposé à la surface du milieu de culture de telle façon que sa diffusion assure un gradient de concentration de cet agent à la surface du milieu de culture.

5. Procédé selon l'une quelconque des revendications 1 à 4, dans lequel la souche utilisée est IFP 903 (ATCC 39.057).

6. Mutant de Clostridium acetobutylicum, ledit mutant portant le numéro IFP 904 (ATCC 39.058).

7. Utilisation d'un mutant obtenu par le procédé de l'une quelconque des revendications 1 à 5, comme agent de fermentation, pour la production d'un mélange de butanol et d'acétone.

8. Utilisation d'un mutant selon la revendication 7, ledit mutant étant IFP 904 (ATCC 39.058).


**Patentansprüche**

1. Verfahren zur Gewinnung von Mutanten von Clostridium acetobutylicum mit hoher Produktivität von n-Butanol und Aceton, sowie einer verbesserten Widerstandsfähigkeit gegenüber n-Butanol, dadurch gekennzeichnet, daß man unter anaeroben Bedingungen eine wäßrige Kultur eines Stammes von Clostridium acetobutylicum auf der Oberfläche eines festen Kulturmilieus, welches n-Butanol enthält, verteilt, ein mutagenes Mittel auf die Oberfläche des Kulturmilieus aufbringt, das erhaltene Milieu den Bedingungen der Entwicklungen dieses Stammes unterwirft und mindestens einen Teil einer Kolonie der gebildeten Mutanten isoliert, wobei die Konzentration des n-Butanols im Kulturmilieu mindestens gleich derjenigen ist, oberhalb der man keine wesentliche Entwicklung des Ausgangsstammes in Abwesenheit des mutagenen Mittels mehr beobachtet, aber geringer als diejenige Konzentration, oberhalb der in Anwesenheit des mutagenen Mittels keine Entwicklungskolonie sich bildet.

2. Verfahren nach Anspruch 1, dadurch gekennzeichnet, daß man als mutagenes Mittel N-Methyl-N'-Nitro-N-Nitrosoguanidin verwendet.

3. Verfahren gemäß Ansprüchen 1 und 2, dadurch gekennzeichnet, daß die isolierte Mutante gereinigt wird, indem man sie erneut mindestens einmal in einem Kulturmilieu ansetzt, welches n-Butanol in einer Konzentration enthält, die höher ist als die Minimalkonzentration, bei welcher der nichtmutierte Stamm sich nicht mehr entwickelt, aber geringer als die Minimalkonzentration, bei der die Mutante sich nicht mehr entwickelt.

4. Verfahren gemäß Ansprüchen 1 bis 3, dadurch gekennzeichnet, daß das mutagene Mittel so auf der Oberfläche des Kulturmilieus verteilt wird, daß seine Verteilung einen Konzentrationsgradienten dieses Mittels auf der Oberfläche des Kulturmilieus gewährleistet.

5. Verfahren gemäß Ansprüchen 1 bis 4, dadurch gekennzeichnet, daß der verwendete Stamm IFP 903 (ATCC 39.057) ist.

6. Mutante von Clostridium acetobutylicum mit der Nr. IFP 904 (ATCC 39.058).

7. Verwendung einer Mutante, die man nach den Verfahren der Ansprüche 1 bis 5 erhalten hat, als Fermentationsmittel für die Gewinnung eines Gemisches von Butanol und Aceton.

8. Verwendung einer Mutante gemäß Anspruch 7, wobei diese Mutante IFP 904 (ATCC 39.058).

**Claims**

1. A process for producing mutants of Clostridium acetobutylicum having a high productivity of n-butanol and acetone and an improved resistance to n-butanol, which comprises, under anaerobic conditions:

spreading out an aqueous Culture of a Clostridium acetobutylicum strain at the surface of a solid culture medium contaning n-butanol,
setting a mutagenic agent at the surface of the culture medium,
subjecting the resultant medium to growth conditions for said strain,
recovering at least a portion of a formed mutant colony, the n-butanol concentration of the culture medium being at least that above which no substantial growth of the starting strain, in the absence of the mutagenic agent, is observed, however lower that the concentration above which, in the presence of the mutagenic agent, no growth colony forms.

. A process according to claim 1, wherein the mutagenic agent is N-methyl N′-nitro N-nitrosoguanidine.

3. A process according to claim 1 or 2, wherein the mutagenic agent is purified by growing it again at least once in a culture medium containing n-butanol at a concentration higher than the minimum concentration at which the original strain (no mutation) no longer grows but lower than the minimum concentration at which the mutant no longer grows.

4. A process according to one of claims 1 to 3, wherein the mutagenic agent is placed at the surface of the culture medium in such a manner that its diffusion produces a concentration gradient at the surface of the culture medium.

5. A process according to any of claims 1 to 4, wherein the strain is IFP 903 (ATCC 39 057).

6. The Clostridium acetobutylicum mutant IFP 904 (ATCC 39 058).

7. The use of a mutant obtained by the process of any of claims 1 to 5, as fermentation agent, to produce a mixture of butanol and acetone.

8. The use of a mutant according to claim 7, said mutant being IFP 904 (ATCC 39 058).